# FASCICULE DE BREVET EUROPEEN

(11) **EP 1 563 828 B1**
(45) Date de publication et mention de la délivrance du brevet: **15.11.2006**
(21) Numéro de dépôt: 05290032.1
(22) Date de dépôt: 06.01.2005
(51) Int. Cl.: A61K 8/49, A61Q 5/10

(54) **Composition tinctoriale comprenant au moins un colorant direct de la famille azo-pyridinio-pyridone et au moins un colorant de synthèse différent, procédé de coloration des fibres kératiniques**
Färbemittel enthaltend mindenstens einen direktziehenden Farbstoff aus der Familie der Azo-pyridinium-pyridone und einen zusätzlichen synthetischen Farbstoff, Verfahren zum Färben von Keratinfasern
Colouring composition comprising at least one direct colouring agent from the azo-pyridinio-pyridone family and at least one additional synthesized colouring agent, dyeing process of keratinuous fibers

(30) Priorité: 07.01.2004 FR 0450040
(43) Date de publication de la demande: 17.08.2005
(73) Titulaire: L'ORÉAL, 75008 Paris (FR)
(72) Inventeur: Kravtchenko, Sylvain, 92600 Asnieres (FR); Lagrange, Alain, 77700 Coupvray (FR); Guerin, Frédéric, 75009 Paris (FR)
(74) Mandataire: Wattremez, Catherine

(56) Documents cités:
- CH-A- 601 432
- GB-A- 2 173 210
- US-A- 5 015 292

## Description

La présente invention a pour objet une composition tinctoriale comprenant à titre de colorant direct, au moins un colorant de la famille des azo-pyridinio-pyridinones, et au moins un colorant de synthèse différent du premier, ainsi qu'un procédé de coloration des fibres kératiniques, notamment humaines, telles que les cheveux, mettant en oeuvre ladite composition.

Il existe essentiellement deux types de coloration des fibres kératiniques, la coloration dite permanente, et la coloration dite semi-permanente.

La première, aussi appelée coloration d'oxydation, consiste à mettre en oeuvre des précurseurs de colorants dits "d'oxydation", qui sont des composés incolores ou faiblement colorés. Une fois mélangés à des produits oxydants, au moment de l'emploi, ces précurseurs conduisent par un processus de condensation oxydative à des composés colorés et colorants. On obtient alors des couleurs très tenaces et puissantes.

En général, on utilise des composés appelés bases d'oxydation, comme par exemple les para-phénylènediamines, les para-aminophénols, etc., que l'on peut associer le cas échéant à au moins un autre composé, appelé coupleur, dans le but de faire varier les nuances de couleurs obtenues.

Si l'on peut obtenir, grâce au nombre important de combinaisons possibles bases / coupleurs, une riche palette de couleurs, on peut être confronté parfois à des problèmes de variation de ténacité ou de sélectivité d'un composé à l'autre, difficiles à maîtriser, entraînant un virage de la couleur.

Enfin, la coloration d'oxydation ne donne pas non plus entière satisfaction si l'on souhaite obtenir des nuances foncées, et plus particulièrement des gris et des noirs.

La seconde voie de coloration, appelée coloration directe, représente une alternative intéressante à la coloration d'oxydation. En effet, elle ne nécessite pas, comme la coloration d'oxydation, d'utiliser d'agent oxydant en milieu alcalin, ce qui contribue à rendre cette méthode de coloration moins agressive que la première pour la fibre ainsi traitée, et donc à moins dégrader cette dernière.

La coloration directe consiste en effet à colorer les cheveux en faisant pénétrer une molécule colorée qui reste de relativement petite taille, par diffusion à l'intérieur du cheveu. Ces composés permettent d'obtenir des montées de couleur chromatiques.

Cependant l'un des inconvénients de la coloration directe est qu'elle peut être considérée trop sélective. De plus, la coloration est peu tenace, notamment aux shampooings.

La présente invention a pour objet de résoudre les problèmes mentionnés ci-dessus, et ainsi de proposer une composition tinctoriale comprenant des colorants directs, permettant d'obtenir des montées de couleur satisfaisantes sur fibres kératiniques, quel que soit leur degré de sensibilisation, permettant d'accéder à des colorations présentant des reflets foncés variés, naturels, très puissants, peu sélectifs et présentant un très bon niveau de ténacité, qui ne virent pas non plus au fil du temps.

En particulier, on peut obtenir des nuances grises et noires très performantes.

Ces buts et d'autres sont atteints par la présente invention qui a donc pour premier objet une composition tinctoriale comprenant, dans un milieu approprié pour la coloration des fibres kératiniques, notamment humaines,
a) au moins un colorant de formule (I) suivante : 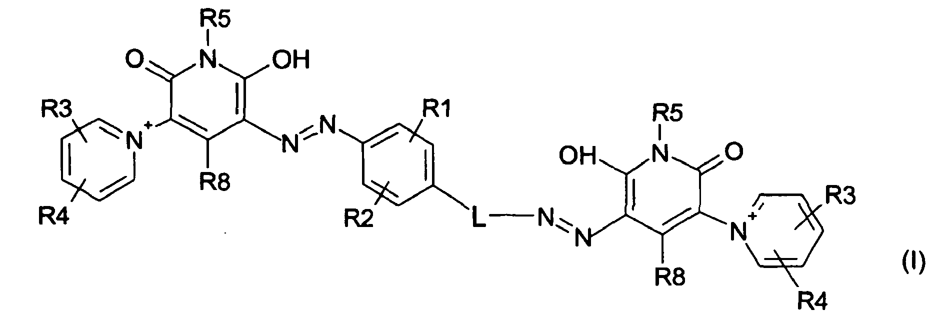 dans laquelle :
   - **R**_{**1**} et **R**_{**2**} représentent, indépendamment l'un de l'autre :
      - un atome d'hydrogène ;
      - un groupe alkyle, linéaire ou ramifié, en C₁-C₄, ledit groupe pouvant être substitué par un ou plusieurs groupes choisis parmi les groupes hydroxyle, amino, halogène, de préférence le chlore, le brome, le fluor, ou alkyle en C₁-C₃;
      - un groupe alcoxy, linéaire ou ramifié, en C₁-C₄ ;
      - un groupe hydroxyle, amino, halogène ou alcoxy, linéaire ou ramifié, en C₁-C₃ ;
   - **R**_{**3**} et **R**_{**4**} représentent, indépendamment l'un de l'autre :
      - un atome d'hydrogène ;
      - un groupe alkyle, linéaire ou ramifié, en C₁-C₄, de préférence un groupe méthyle ;
      - un groupe carboxylique, sous forme acide ou salifiée, un groupe carboxyalkyle, linéaire ou ramifié, en C₁-C₆ ;
      - un groupe alcoxy, linéaire ou ramifié, en C₁-C₆ ;
   - **R**_{**5**} représente :
      - un atome d'hydrogène ;
      - un groupe alkyle, linéaire ou ramifié, en C₁-C₄ ;
      - un groupe alcényle, linéaire ou ramifié, en C₁-C₆, substitué par le groupe (**R**_{**6**}**R**_{**7**}N-)ₙ, n étant égal à 0 ou 1 et lorsque n=1, **R**_{**6**} et **R**_{**7**} représentent indépendamment l'un de l'autre un atome d'hydrogène, un groupe alkyle, linéaire ou ramifié, en C₁-C₆, éventuellement substitué par un ou plusieurs groupes choisis parmi les groupes hydroxyle, amino, halogène, de préférence le chlore, le brome, le fluor, ou alkyle en C₁-C₃;
   - **R**_{**8**} représente :
      - un atome d'hydrogène ;
      - un groupe alkyle, linéaire ou ramifié, en C₁-C₆, et de préférence un groupe méthyle ou éthyle;
   - L représente un groupe divalent de formule **L1** ou **L2** suivantes : 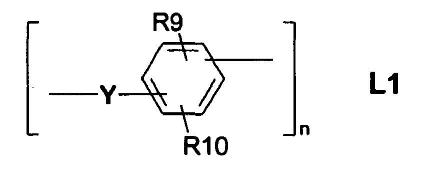 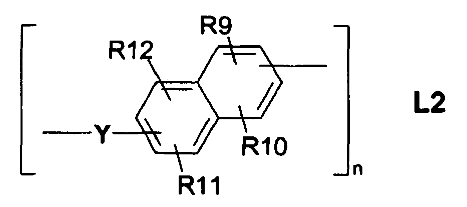
   dans lesquels :
   - **n** est égal à 0 ou 1 ;
   - **R**_{**9**}**, R**_{**10**}**, R**_{**11**} ou **R**_{**12**}**,** représentent, indépendamment l'un de l'autre :
      - un atome d'hydrogène ;
      - un groupe alkyle, linéaire ou ramifié, en C₁-C₆, ledit groupe pouvant être substitué par un ou plusieurs groupes choisis parmi les groupes hydroxyle, amino, halogène, de préférence le chlore, le brome, le fluor ou alcoxy, linéaire ou ramifié, en C₁-C₃ ;
      - un groupe alcoxy, linéaire ou ramifié, en C₁-C₄ ;
      - un groupe hydroxyle, amino, halogène ou alcoxy, linéaire ou ramifié, en C₁-C₃ ;
      - un groupe sulfonique ;
   - **Y** représente un groupe divalent, et
b) au moins un colorant de synthèse différent du composé de formule (I).

Elle a de plus pour objet un procédé de coloration de fibres kératiniques, notamment humaines, et plus particulièrement les cheveux, dans lequel on met en oeuvre la composition tinctoriale précitée, ainsi qu'un dispositif à plusieurs compartiments pour la mise en oeuvre dudit procédé.

Mais d'autres caractéristiques et avantages de la présente invention apparaîtront plus clairement à la lecture de la description qui va suivre.

Le composé de formule (I) entrant comme ingrédient de la composition tinctoriale selon l'invention va tout d'abord être décrit.

Comme indiqué plus haut, ce composé est représenté par la formule (I).

Plus particulièrement, dans cette formule, le groupement Y représente un groupe alkylène en C₁-C₆, éventuellement substitué à chaque extrémité par un groupement carbonyle, ou par un groupement -CO-NH- ou -NH-CO- ; un groupement -CO-NH- ou - NH-CO- ; un groupement carbonyle ou un groupement azoïque.

De préférence, dans la formule (I), n vaut 1 et Y représente un groupement -(CH₂)p-avec p représentant un entier variant de 1 à 4.

Conformément à un mode de réalisation particulier de l'invention, le composé de formule (I) correspond à la formule suivante :

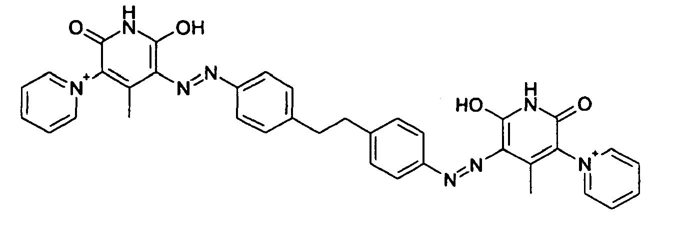

La composition tinctoriale selon la présente invention comprend plus particulièrement de 0,001% à 10% en poids de composé de formule (I), et de préférence de 0,005% à 5% en poids de composé de formule (I), par rapport au poids total de la composition tinctoriale.

Comme mentionné plus haut, la composition tinctoriale comprend, outre le composé qui vient d'être décrit, au moins un colorant synthétique différent du colorant de formule (I).

Par différent, on entend un composé de famille différente de celle des composés de formule (I).

Selon un premier mode de réalisation de l'invention, le colorant synthétique est choisi parmi les colorants directs non ioniques, cationiques, anioniques, amphotères ou zwitterioniques, ou leurs mélanges.

Parmi les colorants directs synthétiques additionnels, on peut citer les colorants acridines, acridones, anthranthrones, anthrapyrimidines, anthraquinones, azines, azos, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines (par exemple les azacarbocyanines, diazacarbocyanines, diazahémicyanines, hémicyanines, tétraazacarbocyanines), diazines, dicétopyrrolopyrroles, dioxazines, diphénylamines, dithiazines, flavanthrones, flavones, fluorindines, formazans, hydrazones, hydroxycétones, indamines, indanthrones, indigoides, indophénols, indoanilines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, styriles, tétrazoliums, thiazines, thioindigo, thiopyronines, xanthènes.

Plus particulièrement, on utilise un ou plusieurs colorants directs synthétiques choisi(s) parmi les colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les molécules fluorescentes.

Parmi les colorants nitro utilisables, on peut citer de manière non limitative les colorants suivants:
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthytamino-2-nitro-4-aminobenzène
- 1-β-hydroxyéthylamino-2-nitro-4-(éthyl)( β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthytamino-5-nitrobenzène
- 1,2-Bis-β-hydroxyéthytamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1 -Méthoxy-2-β-hydroxyéthylamino-5-nitrobenzène
- 1-β-hydroxyéthyloxy-3-méthylamino-4-nitrobenzène
- 1-βγ-dihydroxypropyloxy-3-méthylamino-4-nitrobenzène
- 1-β-hydroxyéthylamino-4-β,γ-dihydroxypropyloxy-2-nitrobenzène
- 1-βγ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants azoïques utilisables, on peut citer les colorants azoïques cationiques décrits dans les demandes de brevets WO 95/15144, WO 95/01772 et EP 714954.

On peut également citer parmi les colorants azoïques, ceux décrits dans le COLOUR INDEX INTERNATIONAL 3^{e} édition comme : Disperse Red 17, Acid Yellow 9 , Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants quinoniques, on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants :
- 1-N-méthylmorpholiniumpropylamino-4-hydroxyanthraq uinone
- 1 -Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(βγ-dihydroxypropylamino)-anthraquinone.

Parmi les colorants aziniques, on peut citer les composés suivants : Basic Blue 17 et Basic Red 2.

Parmi les colorants indoaminiques utilisables, on peut citer les composés suivants :
- 2-(β-hydroxyéthlyamino-5-[bis-([β-4'-hydroxyéthyl)amino]anilino-1,4-benzoquinone
- 2-(β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

On peut aussi citer les colorants décrits dans les documents US 5,888,252, EP 1 133 975, WO 03/029359, EP 860 636, WO 95/01772, WO 95/15144, EP 714954. On peut aussi citer ceux cités dans l'encyclopédie "The chemistry of synthetic dye" de K. VENKATARAMAN, 1952, Academic press vol 1 à 7, dans l'encyclopédie "Kirk Othmer" "Chemical technology", chapitre "dyes and Dye intermediate", 1993, Wiley and sons, et dans divers chapitre de l'encyclopédie "ULLMANN's ENCYCLOPEDIA of Industrial chemistry" 7th édition, Wiley and sons.

Selon ce mode de réalisation de l'invention, la teneur en colorant(s) direct(s) synthétique(s) autre(s) que les composés de formule (I) variant entre 0,001 et 10 % en poids par rapport au poids total de la composition, de préférence comprise entre 0,005 et 5 % en poids par rapport au poids total de la composition.

Selon un autre mode de réalisation de l'invention, le colorant de synthèse est choisi parmi les bases d'oxydation éventuellement associées à au moins un coupleur.

A titre d'exemples de bases d'oxydation, on peut citer notamment les paraphénylènediamines, les bases doubles, les para-aminophénols, les ortho aminophénols et les bases d'oxydation hétérocycliques, seules ou en mélange, ainsi que leurs sels d'addition avec un acide.

Parmi les paraphénylènediamines utilisables, on peut notamment citer les composés de formule (II) suivante et leurs sels d'addition avec un acide : 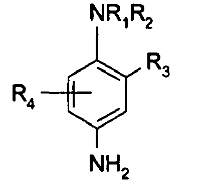 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les paraphénylènediamines de formule (II) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènèdiamine, la 2-β-acétylaminoéthyloxy paraphénylènediamine, la N-(β-méthoxyéthyl) paraphénylènediamine, et leurs sels d'addition avec un acide.

En ce qui concerne les bases doubles, ces composés comportent au moins deux noyaux aromatiques sur lesquels sont portés des groupements amino et/ou hydroxyle, reliés par un bras de liaison.

Parmi les bases doubles utilisables à titre de bases d'oxydation dans les compositions tinctoriales conformes à l'invention, on peut notamment citer les composés répondant à la formule (III) suivante, et leurs sels d'addition avec un acide : 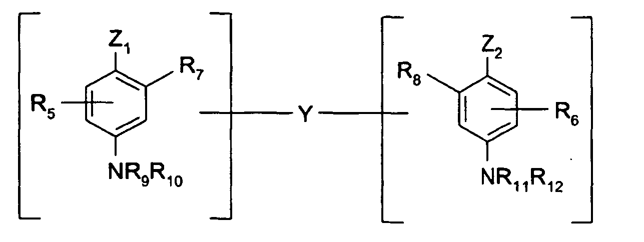 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (III) ne comportent qu'un seul bras de liaison Y par molécule.

Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols utilisables à titre de bases d'oxydation, on peut notamment citer les composés répondant à la formule (IV) suivante, et leurs sels d'addition avec un acide : 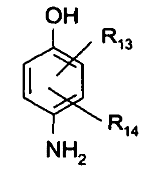 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

A titre de para-aminophénols on peut aussi citer le 4 amino,6 [(5'-amino-2' hydroxy 3'méthylphényl)méthyl] 2 méthylphenol et le bis (5 amino 2'hydroxyphényl méthane) et leurs sels d'addition avec un acide.

Parmi les para-aminophénols de formule (IV) ci-dessus, on peut plus particulièrement citer le para-aminophénol, le 4-amino 3-chloro phénol, le 4-amino 2-chloro phénol, le 2,6-dichloro 4-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (V) suivante, leurs sels d'addition avec un acide
ou avec une base et leurs formes tautomères, lorsqu'il existe un équilibre tautomérique : 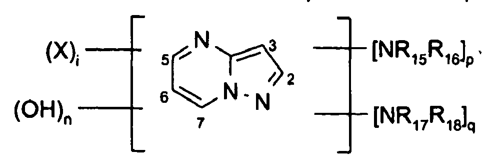 dans laquelle :
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C₄)alkyl ou di-[hydroxy(C₁-C₄ )alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ; p vaut 0 ou 1 ; q vaut 0 ou 1 ; n vaut 0 ou 1 ; sous réserve que la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Parmi les pyrazolo-[1,5-a]-pyrimidines de formule (V) ci-dessus on peut notamment citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol ; le 2-(7-amino pyrazolo-[1,5-a]-pyrimidin-3-ylamino)-éthanol ; le 2-[(3-Amino-pyrazolo[1,5-a)pyrimidin-7-yl)-(2-hydroxy-éthyl)-aminoj-éthanol ; le 2-[(7-Amino-pyrazolo [1,5-a]pyrimidin-3-yl)-(2-hydroxy-éthyl)-amino]-éthanol ; la 5,6-diméthyl pyrazolo-[1,5-a]-pyrimidine-3,7-diamine ; la 2,6-diméthyl pyrazolo-[1,5-a]-pyrimidine-3,7-diamine ; la 2, 5, N 7, N 7-tetraméthyl pyrazolo-[1,5-a]-pyrimidine-3,7-diamine ; et leurs sels d'addition et leurs formes tautomères, lorsqu'il existe un équilibre tautomérique.

Lorsque la composition tinctoriale comprend une ou plusieurs bases d'oxydation, leur teneur représente habituellement de 0,0005 à 12 % en poids, de préférence de 0,005 à 8 % en poids, par rapport au poids total de la composition tinctoriale.

La composition tinctoriale peut de plus comprendre au moins un coupleur associé à une ou plusieurs bases d'oxydation.

Ces coupleurs sont avantageusement choisis parmi ceux classiquement utilisés dans les compositions de teinture d'oxydation, c'est-à-dire des méta-phénylènediamines, des méta-aminophénols, des métadiphénols, des coupleurs hétérocycliques, et leurs sels d'addition avec un acide.

Ces coupleurs peuvent notamment être choisis parmi le 2-méthy)-5-amino-phéno), le 5-N-([i-hydroxyéthyl)-amino-2-méthyl-phénol, le 3-amino-phénol, le , 1,3-dihydroxy-benzène, le 1,3-dihydroxy-2-méthyl-benzène, le 4-chloro-1,3-dihydroxy-benzène, le 2,4-diamino-1-([i-hydroxyéthyloxy)-benzène, le 2-amino-4-((3-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, le 2,6-diméthyl- pyrazoto-[1,5-b]-1,2,4-triazole, le 2,6-diméthyl-[3,2-c]-1,2,4-triazole, le 6-méthyl-pyrazolo-[1,5-a]-benzimidazole, et leurs sels d'addition avec un acide.

Lorsqu'ils sont présents dans la composition colorante, la teneur en coupleur représente en général 0,0001 à 10 % en poids, et de préférence de 0,005 à 5 % en poids par rapport au poids de la composition tinctoriale.

D'une manière générale, les sels d'addition avec un acide utilisables dans le cadre des compositions tinctoriales de l'invention sont notamment choisis parmi les chlorhydrates, les bromhydrates, les sulfates et les tartrates, les lactates et les acétates.

La composition selon l'invention peut également comprendre divers adjuvants utilisés classiquement, tels que des agents tensioactifs anioniques, cationiques, non ioniques, amphotères, zwittérioniques ou leurs mélanges, des polymères anioniques, cationiques, non ioniques, amphotères, zwittérioniques, des agents épaississants minéraux, des polymères épaississants, associatifs ou non, agents antioxydants, des agents séquestrants, des agents de pénétration, des parfums, des tampons, des agents dispersants, des agents de conditionnement tels que par exemple des cations, des silicones volatiles ou non volatiles, modifiées ou non modifiées, des polymères amphiphiles cationiques, des agents filmogènes, des céramides, des vitamines ou provitamines, des agents conservateurs, des agents stabilisants, des agents opacifiants ou matifiants comme le dioxyde de titane, des charges minérales, comme les argiles, les silices notamment pyrogénées à caractère hydrophile ou hydrophobe, des polymères liants tels que la vinylpyrrolidone, des filtres solaires, etc.

Les adjuvants cités ci-dessus sont en général présents en quantité comprise pour chacun d'eux entre 0,01 et 20% en poids par rapport au poids de la composition tinctoriale.

Le milieu de la composition tinctoriale est un milieu cosmétiquement acceptable.

Il est constitué, de manière avantageuse, par de l'eau ou par un mélange d'eau et d'un ou plusieurs solvants organiques.

A titre de solvant organique, on peut par exemple citer les monoalcools, linéaire ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.

Les solvants organiques décrits ci-dessus, s'ils sont présents, représentent habituellement de 1 à 40 % en poids, plus préférentiellement de 5 à 30 % en poids, par rapport au poids total de la composition tinctoriale.

Notons que la composition selon l'invention peut se présenter sous des formes galéniques diverses, telles qu'une lotion, une crème, un gel ou sous tout autre forme appropriée pour réaliser une teinture des fibres kératiniques. Elle peut également être conditionnée sous pression en flacon aérosol en présence d'un agent propulseur et former une mousse.

La composition tinctoriale mise en oeuvre dans le cadre de l'invention peut éventuellement comprendre au moins un agent oxydant.

Habituellement, l'agent oxydant est choisi parmi le peroxyde d'hydrogène, les peroxydes de métaux alcalins ou alcalino-terreux, comme le sodium, le potassium, le magnésium ; le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges. L'agent oxydant peut aussi être choisi parmi les enzymes telles que les peroxydases et les oxydoréductases à deux ou à quatre électrons. De préférence, l'agent oxydant est le peroxyde d'hydrogène.

S'il est présent, la teneur en agent oxydant représente 0,001 et 20% en poids par rapport au poids total de la composition tinctoriale.

Si la composition mise en oeuvre dans le procédé selon l'invention comprend un agent oxydant, alors cette composition, appelée aussi composition prête à l'emploi, résulte plus particulièrement du mélange de la composition décrite auparavant dépourvue d'agent oxydant, avec une composition comprenant au moins un tel agent (cette composition est alors plus particulièrement appelée composition oxydante). Dans ce cas, le mélange est réalisé avant l'application de la composition prête à l'emploi sur les fibres à traiter.

Le milieu de la composition oxydante précitée est avantageusement de l'eau ou un mélange d'eau et de solvant organique.

Enfin, la composition oxydante peut comprendre les additifs usuels dans le domaine, comme des agents tensioactifs, des agents épaississants, des agents antioxydants, des parfums, des agents dispersants, des agents de conditionnement, des agents séquestrants, des agents conservateurs, etc.

Précisons que les listes de solvants et d'additifs, de même que leurs teneurs, indiquées dans le cadre de la description de la composition tinctoriale restent valables et l'on peut s'y reporter.

Le pH de la composition tinctoriale mise en oeuvre dans le procédé selon l'invention varie généralement de 3 à 12, plus particulièrement de 5 et 11, et de préférence de 6 à 8,5.

Il peut être ajusté à la valeur désirée au moyen d'agents acidifiants ou alcalinisants.

Parmi les agents acidifiants, on peut citer, à titre d'exemples, les acides minéraux ou organiques comme l'acide chlorhydrique, l'acide orthophosphorique, l'acide sulfurique, l'acide acétique.

Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 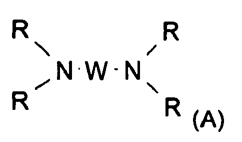 dans laquelle W est un reste propylène éventuellement substitué par un groupement hydroxyle ou un radical alkyle en C₁-C₆ ; les radicaux R, identiques ou différents, représentent un atome d'hydrogène, un radical alkyle en C₁-C₆ ou hydroxyalkyle en C₁-C₆.

Un autre objet de la présente invention est constitué par un procédé de coloration des fibres kératiniques, notamment humaines, dans lequel on met en contact les fibres kératiniques, sèches ou humides, avec une composition tinctoriale selon l'invention, pendant une durée suffisante pour développer la coloration souhaitée.

Plus particulièrement, le procédé selon l'invention consiste à effectuer les étapes suivantes :
a) on met en contact les fibres, sèches ou humides, avec la composition qui vient d'être décrite, pendant une durée suffisante pour le développement de la coloration,
b) on rince éventuellement les fibres,
c) éventuellement lave les fibres et on les rince,
d) on sèche les fibres ou on les laisse sécher.

Selon une première variante de l'invention, la composition appliquée sur les fibres ne comprend pas d'agent oxydant.

Cette variante est particulièrement appropriée lorsque la composition ne comprend que le ou les composés de formule (I) et au moins un colorant de synthèse choisi parmi les colorants directs.

Dans le cadre de cette variante, il est envisageable de ne pas rincer ni laver au shampooing, les fibres une fois traitées.

De préférence, on procédera toutefois à un tel rinçage, et éventuellement au lavage des fibres au moyen d'un shampooing notamment.

Selon une deuxième variante, la composition appliquée sur les fibres comprend au moins un agent oxydant.

Cette variante du procédé est appropriée pour tout type de composition tinctoriale, que celle-ci comprenne ou non, parmi les colorants de synthèse, une ou plusieurs bases d'oxydation éventuellement associées à au moins un coupleur.

Selon cette deuxième variante, une première possibilité consiste à préparer la composition destinée à être appliquée sur les fibres, soit la composition prête à l'emploi, extemporanément avant l'application, par mélange d'une ou plusieurs compositions tinctoriales, dépourvues d'agent oxydant, dont l'une au moins comprend au moins un composé de formule (I), au moins une autre comprend au moins un colorant de synthèse différent du composé de formule (I) (ces deux compositions pouvant être les mêmes), avec une composition comprenant au moins un agent oxydant.

Ainsi, on peut envisager par exemple, de procéder à partir d'une composition tinctoriale comprenant à la fois le composé de formule (I) et le colorant de synthèse différent du composé de formule (I), ou bien à partir de deux compositions tinctoriales ou plus, l'une au moins comprenant le composé de formule (I) et une autre au moins comprenant le ou les colorants de synthèse différent du composé de formule (I).

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants, alcalinisants ou tampons, habituellement utilisés en coloration des fibres kératiniques et tels que définis précédemment.

Selon cette possibilité, il peut être avantageux de stocker sous forme séparée, d'une part, la ou les compositions tinctoriales dépourvues d'agent oxydant, et d'autre part, une composition oxydante.

Toujours selon la deuxième variante du procédé, on peut envisager appliquer successivement dans un ordre ou dans l'autre, la ou les compositions tinctoriales et la composition comprenant au moins un agent oxydant, avec ou sans rinçage intermédiaire, bien encore consiste à appliquer ces deux compositions simultanément.

La durée nécessaire au développement de la coloration est habituellement de l'ordre de 1 à 60 minutes et de préférence de 5 à 45 minutes, et de manière encore plus avantageuse de 10 à 20 minutes.

Par ailleurs, de manière classique l'étape a) du procédé est effectuée à une température varie de la température ambiante (environ 15 à 25°C) et 80°C, de préférence à une température allant de 25 et 55°C.

Une fois l'étape a) terminée, on peut éventuellement rincer les fibres (étape b), et éventuellement les laver avec un shampooing puis les rincer. De préférence, on rince et on lave les fibres avec un shampooing.

Enfin, on sèche lesdites fibres ou bien encore on les laisse sécher, par exemple entre 20 et 220°C.

Enfin, la présente invention a pour objet un dispositif à plusieurs compartiments pour la mise en oeuvre du procédé qui vient d'être décrit et qui met en oeuvre une composition oxydante.

Plus spécialement, ce dispositif comprend un compartiment comprenant une composition tinctoriale comprenant au moins un composé de formule (I) et éventuellement au moins un colorant de synthèse différent du composé de formule (I) ; éventuellement au moins un deuxième compartiment comprenant une composition tinctoriale comprenant au moins un colorant de synthèse différent du composé de formule (I), et au moins un troisième compartiment comprenant une composition comprenant au moins un agent oxydant.

## Revendications

1. Composition tinctoriale comprenant, dans un milieu approprié pour la coloration des fibres kératiniques , notamment humaines,
a) au moins un colorant de formule (I) suivante : 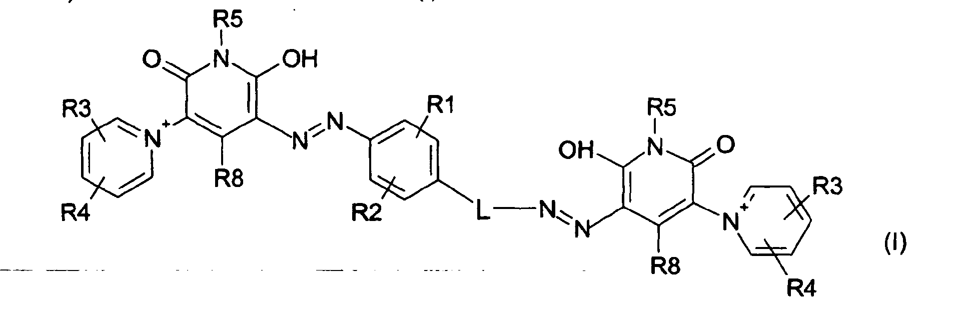 dans laquelle :
- **R**_{**1**} et **R**_{**2**} représentent, indépendamment l'un de l'autre :
- un atome d'hydrogène ;
- un groupe alkyle, linéaire ou ramifié, en C₁-C₄, ledit groupe pouvant être substitué par un ou plusieurs groupes choisis parmi les groupes hydroxyle, amino, halogène, de préférence le chlore, le brome, le fluor, ou alkyle en C₁₋C₃;
- un groupe alcoxy, linéaire ou ramifié, en C₁-C₄ ;
- un groupe hydroxyle, amino, halogène ou alcoxy, linéaire ou ramifié, en C₁-C₃ ;
- **R**_{**3**} et **R**_{**4**} représentent, indépendamment l'un de l'autre :
- un atome d'hydrogène ;
- un groupe alkyle, linéaire ou ramifié, en C₁-C₄, de préférence un groupe méthyle ;
- un groupe carboxylique, sous forme acide ou salifiée, un groupe carboxyalkyle, linéaire ou ramifié, en C₁-C₆ ;
- un groupe alcoxy, linéaire ou ramifié, en C₁-C₆ ;
- **R**_{**5**} représente :
- un atome d'hydrogène ;
- un groupe alkyle, linéaire ou ramifié, en C₁-C₄ ;
- un groupe alcényle, linéaire ou ramifié, en C₁-C₆, substitué par le groupe **(R**_{**6**}**R**_{**7**}N-)ₙ, n étant égal à 0 ou 1 et lorsque n=1, **R**_{**6**} et **R**_{**7**} représentent indépendamment l'un de l'autre un atome d'hydrogène, un groupe alkyle, linéaire ou ramifié, en C₁-C₆, éventuellement substitué par un ou plusieurs groupes choisis parmi les groupes hydroxyle, amino, halogène, de préférence le chlore, le brome, le fluor, ou alkyle en C₁-C₃ ;
- **R**_{**8**} représente :
- un atome d'hydrogène ;
- un groupe alkyle, linéaire ou ramifié, en C₁-C₆, et de préférence un groupe méthyle ou éthyle;
- **L** représente un groupe divalent de formule **L1** ou **L2** suivantes : 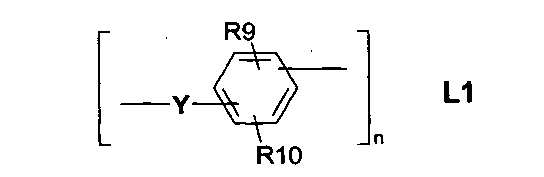 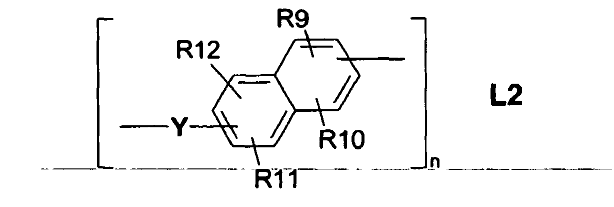
dans lesquels :
- **n** est égal à 0 ou 1 ;
- **R**_{**9**}**, R**_{**10**}**, R**_{**11**} ou **R**_{**12**}**,** représentent, indépendamment l'un de l'autre :
- un atome d'hydrogène ;
- un groupe alkyle, linéaire ou ramifié, en C₁-C₆, ledit groupe pouvant être substitué par un ou plusieurs groupes choisis parmi les groupes hydroxyle, amino, halogène, de préférence le chlore, le brome, le fluor ou alcoxy, linéaire ou ramifié, en C₁-C₃ ;
- un groupe alcoxy, linéaire ou ramifié, en C₁-C₄ ;
- un groupe hydroxyle, amino, halogène ou alcoxy, linéaire ou ramifié, en C₁-C₃ ;
- un groupe sulfonique ;
- **Y** représente un groupe divalent, et
b) au moins un colorant de synthèse différent du composé de formule (I).

2. Composition tinctoriale selon la revendication précédente, **caractérisée en ce que**, dans la formule (I), le groupement Y représente un groupe alkylène de 1 à 4 atomes de carbone, éventuellement substitué à chaque extrémité un groupement carbonyle, ou par un groupement -CO-NH- ou -NH-CO- ; un groupement -CO-NH- ou -NH-CO- ; un groupement carbonyle ou un groupement azoïque.

3. Composition tinctoriale selon la revendication 2, **caractérisée en ce que** n vaut 1, Y représente -(CH₂)ₚ- avec p représentant un entier compris entre 1 et 4.

4. Composition tinctoriale selon l'une quelconque des revendications précédentes, **caractérisée en ce que** le composé de formule (I) correspond à un composé de formule suivante :

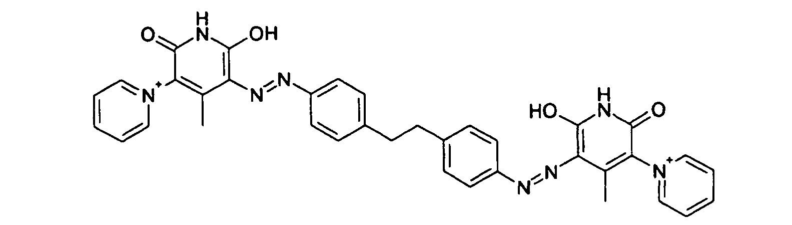

5. Composition tinctoriale selon l'une quelconque des revendications précédentes, **caractérisée en ce que** la teneur en composé de formule (I) est variant de 0,01 à 10 % en poids par rapport au poids total de la composition tinctoriale, de préférence variant de 0,005 à 5 % en poids par rapport au poids total de la composition tinctoriale.

6. Composition tinctoriale selon l'une quelconque des revendications précédentes, **caractérisée en ce que** le colorant de synthèse différent du composé de formule (I) est choisi parmi les colorants directs non ioniques, cationiques, anioniques, amphotères ou zwitterioniques, ou leurs mélanges.

7. Composition tinctoriale selon la revendication précédente, **caractérisée en ce que** le colorant direct de synthèse est choisi parmi les colorants acridines, acridones, anthranthrones, anthrapyrimidines, anthraquinones, azines, azos, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines (azacarbocyanine, diazacarbocyanine, diazahémicyanine, hémicyanine, tétraazacarbocyanine), diazines, dicétopyrrolopyrroles, dioxazines, diphénylamines, dithiazines, flavanthrones, flavones, fluorindines, formazans, hydrazones, hydroxycétones, indamines, indanthrones, indigoides, indophénols, indoanilines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, styriles, tétrazoliums, thiazines, thioindigo, thiopyronines, xanthènes.

8. Composition tinctoriale selon l'une quelconque des revendications 6 ou 7, **caractérisée en ce que** le colorant direct de synthèse est choisi parmi les colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les molécules fluorescentes.

9. Composition tinctoriale selon l'une quelconque des revendications précédentes, **caractérisée en ce que** la teneur en colorant direct de synthèse, différent du composé de formule (I), varie de 0,001 à 10 % en poids par rapport au poids total de la composition tinctoriale, de préférence de 0,005 à 5 % en poids par rapport au poids total de la composition tinctoriale.

10. Composition tinctoriale selon l'une quelconque des revendications précédentes, **caractérisée en ce que** le colorant de synthèse est choisi parmi les bases d'oxydation éventuellement associées à au moins un coupleur.

11. Composition tinctoriale selon la revendication précédente, **caractérisée en ce que** la base d'oxydation est choisie parmi les paraphénylènediamines, les bases doubles, les para-aminophénols, les ortho aminophénols et les bases d'oxydation hétérocycliques, seules ou en mélange, ainsi que leurs sels d'addition avec un acide.

12. Composition tinctoriale selon l'une des revendications 10 ou 11, **caractérisée en ce que** la teneur en base d'oxydation varie de 0,0005 à 12 % en poids par rapport au poids total de la composition tinctoriale, de préférence de 0,005 à 8 % en poids par rapport au poids total de la composition tinctoriale.

13. Composition tinctoriale selon l'une quelconque des revendications 10 à 12, **caractérisée en ce que** le coupleur est choisi parmi les méta-phénylènediamines, les méta-aminophénols, les métadiphénols, les coupleurs hétérocycliques, seules ou en mélange, ainsi que leurs sels d'addition avec un acide.

14. Composition tinctoriale selon l'une quelconque des revendications précédentes, **caractérisée en ce qu**'elle comprend au moins un agent oxydant.

15. Composition tinctoriale selon la revendication précédente, **caractérisée en ce que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur respectif.

16. Procédé de coloration des fibres kératiniques, notamment humaines, **caractérisé en ce que** l'on met en contact les fibres kératiniques, sèches ou humides, avec une composition tinctoriale selon l'une quelconque des revendications 1 à 15, pendant une durée suffisante pour développer la coloration souhaitée.

17. Procédé de coloration selon la revendication précédente, **caractérisé en ce que** l'on prépare la composition extemporanément avant l'application, par mélange d'une composition comprenant au moins un composé de formule (I) et au moins un colorant de synthèse différent du composé de formule (I), avec une composition comprenant au moins un agent oxydant.

18. Procédé de coloration selon la revendication 17, **caractérisé en ce que** l'on prépare la composition extemporanément avant l'application, par mélange d'une composition comprenant au moins un composé de formule (I), d'une composition comprenant au moins un colorant de synthèse différent du composé de formule (I), avec une composition comprenant au moins un agent oxydant.

19. Procédé selon l'une quelconque des revendications 16 à 18, **caractérisée en ce que** l'on applique successivement dans un ordre ou dans l'autre, la ou les compositions tinctoriales et la composition comprenant au moins un agent oxydant.

20. Dispositif à plusieurs compartiments pour la mise en oeuvre du procédé selon l'une quelconque des revendications 16 à 19, **caractérisé en ce qu**'il comprend un compartiment comprenant une composition tinctoriale comprenant au moins un composé de formule (I) et éventuellement au moins un colorant de synthèse différent du composé de formule (I) ; éventuellement au moins un deuxième compartiment comprenant une composition tinctoriale comprenant au moins un colorant de synthèse différent du composé de formule (I), et au moins un troisième compartiment comprenant une composition comprenant au moins un agent oxydant.

## Claims

1. Dye composition comprising, in a medium that is suitable for dyeing keratin fibres, especially human keratin fibres,
a) at least one dye of formula (I) below: 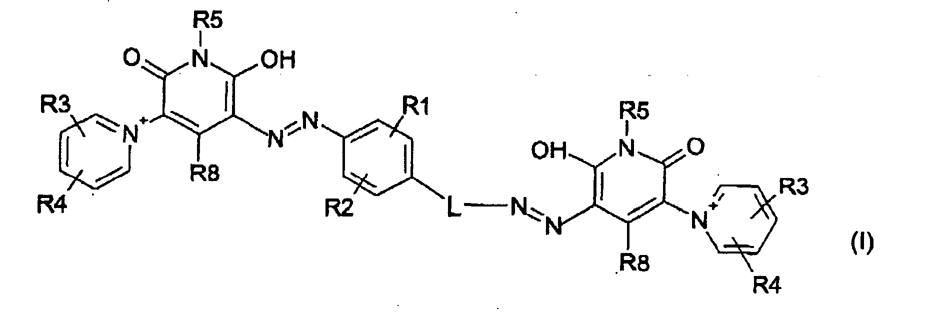 in which:
- R₁ and R₂ represent, independently of each other:
- a hydrogen atom;
- a linear or branched C₁-C₄ alkyl group, the said group possibly being substituted with one or more groups chosen from hydroxyl, amino, halogen, preferably chlorine, bromine or fluorine, and C₁-C₃ alkyl groups;
- a linear or branched C₁-C₄ alkoxy group;
- a hydroxyl, amino, halogen or linear or branched C₁-C₃ alkoxy group;
- R₃ and R₄ represent, independently of each other:
- a hydrogen atom;
- a linear or branched C₁-C₄ alkyl group, preferably a methyl group;
- a carboxylic group, in acid or salified form, or a linear or branched, C₁-C₆ carboxyalkyl group;
- a linear or branched C₁-C₆ alkoxy group;
- R₅ represents:
- a hydrogen atom;
- a linear or branched C₁-C₄ alkyl group;
- a linear or branched C₁-C₆ alkenyl group, substituted with the group (R₆R₇N-)ₙ, n being equal to 0 or 1 and, when n = 1, R₆ and R₇ represent, independently of each other, a hydrogen atom or a linear or branched C₁-C₆ alkyl group, optionally substituted with one or more groups chosen from hydroxyl, amino, halogen, preferably chlorine, bromine or fluorine, and C₁-C₃ alkyl groups;
- R₈ represents:
- a hydrogen atom;
- a linear or branched C₁-C₆ alkyl group and preferably a methyl or ethyl group;
- L represents a divalent group of formula L1 or L2 below: 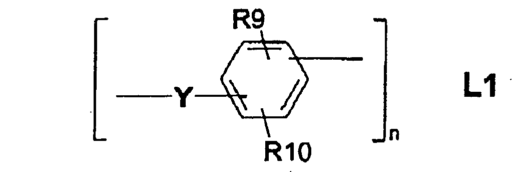 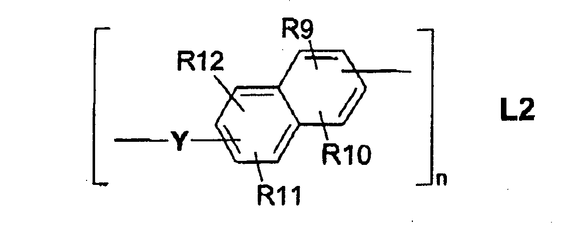
in which:
- n is equal to 0 or 1;
- R₉, R₁₀, R₁₁ or R₁₂ represent, independently of each other:
- a hydrogen atom;
- a linear or branched C₁-C₆ alkyl group, the said group possibly being substituted with one or more groups chosen from hydroxyl, amino, halogen, preferably chlorine, bromine or fluorine, and linear or branched C₁-C₃ alkoxy groups;
- a linear or branched C₁-C₄ alkoxy group;
- a hydroxyl, amino, halogen or linear or branched C₁-C₃ alkoxy group;
- a sulfonic group;
- Y represents a divalent group, and
b) at least one synthetic dye different from the compound of formula (I).

2. Dye composition according to the preceding claim, **characterized in that**, in formula (I), the group Y represents an alkylene group of 1 to 4 carbon atoms, optionally substituted at each end with a carbonyl group, or with a -CO-NH- or -NH-CO-group; a -CO-NH- or -NH-CO- group; a carbonyl group or an azo group.

3. Dye composition according to Claim 2, **characterized in that** n is 1 and Y represents -(CH₂)ₚ- with p representing an integer between 1 and 4.

4. Dye composition according to any one of the preceding claims, **characterized in that** the compound of formula (I) corresponds to a compound of the following formula:

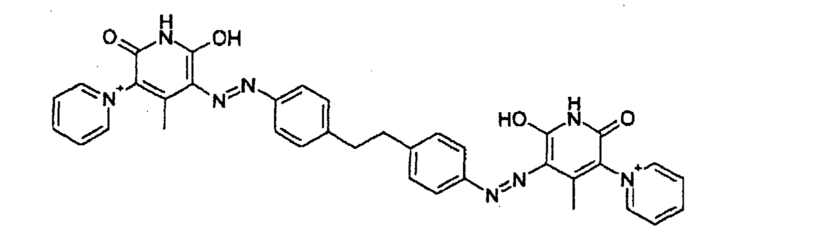

5. Dye composition according to any one of the preceding claims, **characterized in that** the content of compound of formula (I) ranges from 0.01% to 10% by weight relative to the total weight of the dye composition and preferably ranges from 0.005% to 5% by weight relative to the total weight of the dye composition.

6. Dye composition according to any one of the preceding claims, **characterized in that** the synthetic dye different from the compound of formula (I) is chosen from nonionic, cationic, anionic, amphoteric and zwitterionic direct dyes, or mixtures thereof.

7. Dye composition according to the preceding claim, **characterized in that** the synthetic direct dye is chosen from acridine, acridone, anthranthrone, anthrapyrimidine, anthraquinone, azine, azo, azomethine, benzanthrone, benzimidazole, benzimidazolone, benzindole, benzoxazole, benzopyran, benzothiazole, benzoquinone, bis-azine, bis-isoindoline, carboxanilide, coumarin, cyanin (azacarbocyanin, diazacarbocyanin, diazahemicyanin, hemicyanin and tetraazacarbocyanin), diazine, diketopyrrolopyrrole, dioxazine, diphenylamine, dithiazine, flavanthrone, flavone, fluorindine, formazan, hydrazone, hydroxy ketone, indamine, indanthrone, indigoid, indophenol, indoaniline, isoindoline, isoindolinone, isoviolanthrone, lactone, methine, naphthalimide, naphthanilide, naphtholactam, naphthoquinone, nitro, oxadiazole, oxazine, perilone, perinone, perylene, phenazine, phenothiazine, phthalocyanin, polyene/carotenoid, porphyrin, pyranthrone, pyrazolanthrone, pyrazolone, pyrimidinoanthrone, pyronine, quinacridone, quinoline, quinophthalone, squarane, stilbene, styryl, tetrazolium, thiazine, thioindigo, thiopyronine and xanthene dyes.

8. Dye composition according to either of Claims 6 and 7, **characterized in that** the synthetic direct dye is chosen from neutral, acidic or cationic nitrobenzene direct dyes, neutral, acidic or cationic azo direct dyes, neutral, acidic or cationic quinone, and in particular anthraquinone direct dyes, azine, triarylmethane and indoamine direct dyes, methines, styryls, porphyrins, metalloporphyrins, phthalocyanins, methine cyanins, and fluorescent molecules.

9. Dye composition according to any one of the preceding claims, **characterized in that** the content of synthetic direct dye, different from the compound of formula (I), ranges from 0.001% to 10% by weight relative to the total weight of the dye composition, and preferably from 0.005% to 5% by weight relative to the total weight of the dye composition.

10. Dye composition according to any one of the preceding claims, **characterized in that** the synthetic dye is chosen from oxidation bases optionally combined with at least one coupler.

11. Dye composition according to the preceding claim, **characterized in that** the oxidation base is chosen from para-phenylenediamines, double bases, para-aminophenols, ortho-aminophenols and heterocyclic oxidation bases, alone or as a mixture, and also the addition salts thereof with an acid.

12. Dye composition according to either of Claims 10 and 11, **characterized in that** the content of oxidation base ranges from 0.0005% to 12% by weight relative to the total weight of the dye composition, and preferably from 0.005% to 8% by weight relative to the total weight of the dye composition.

13. Dye composition according to any one of Claims 10 to 12, **characterized in that** the coupler is chosen from meta-phenylenediamines, meta-aminophenols, meta-diphenols and heterocyclic couplers, alone or as a mixture, and also the addition salts thereof with an acid.

14. Dye composition according to any one of the preceding claims, **characterized in that** it comprises at least one oxidizing agent.

15. Dye composition according to the preceding claim, **characterized in that** the oxidizing agent is chosen from hydrogen peroxide, urea peroxide, alkali metal bromates or ferricyanides, persalts, redox enzymes such as laccases, peroxidases and two-electron oxidoreductases, where appropriate in the presence of the respective donor thereof.

16. Process for dyeing keratin fibres, especially human keratin fibres, **characterized in that** the wet or dry keratin fibres are placed in contact with a dye composition according to any one of Claims 1 to 15, for a time that is sufficient to develop the desired coloration.

17. Dyeing process according to the preceding claim, **characterized in that** the composition is prepared extemporaneously before application, by mixing a composition comprising at least one compound of formula (I) and at least one synthetic dye different from the compound of formula (I), with a composition comprising at least one oxidizing agent.

18. Dyeing process according to Claim 17, **characterized in that** the composition is prepared extemporaneously before application, by mixing a composition comprising at least one compound of formula (I) and a composition comprising at least one synthetic dye different from the compound of formula (I), with a composition comprising at least one oxidizing agent.

19. Process according to any one of Claims 16 to 18, **characterized in that** the dye composition(s) and the composition comprising at least one oxidizing agent are applied successively in either order.

20. Multi-compartment device for implementing the process according to any one of Claims 16 to 19, **characterized in that** it comprises a compartment comprising a dye composition comprising at least one compound of formula (I) and optionally at least one synthetic dye different from the compound of formula (I); optionally at least one second compartment comprising a dye composition comprising at least one synthetic dye different from the compound of formula (I), and at least one third compartment comprising a composition comprising at least one oxidizing agent.

## Patentansprüche

1. Farbmittelzusammensetzung, die in einem zum Färben von Keratinfasern und insbesondere menschlichen Keratinfasern geeigneten Medium enthält:
a) mindestens einen Farbstoff der folgenden Formel (I): 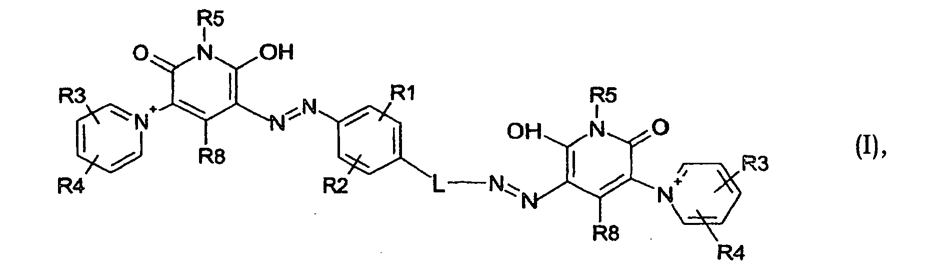 worin bedeuten:
- R₁ und R₂ unabhängig voneinander:
- ein Wasserstoffatom;
- eine geradkettige oder verzweigte C₁₋₄-Alkylgruppe, wobei die Gruppe mit einer oder mehreren Gruppen substituiert sein kann, die unter den Gruppen Hydroxy, Amino, Halogen, vorzugsweise Chlor, Brom oder Fluor, oder C₁₋₃-Alkyl ausgewählt sind;
- eine geradkettige oder verzweigte C₁₋₄-Alkoxygruppe;
- eine Hydroxygruppe, Aminogruppe, ein Halogen oder eine geradkettige oder verzweigte C₁₋₃-Alkoxygruppe;
- R₃ und R₄ unabhängig voneinander:
- ein Wasserstoffatom;
- eine geradkettige oder verzweigte C₁₋₄-Alkylgruppe und vorzugsweise Methyl;
- eine Carboxygruppe in Form der Säure oder als Salz, eine geradkettige oder verzweigte C₁₋₆-Carboxyalkylgruppe;
- eine geradkettige oder verzweigte C₁₋₆-Alkoxygruppe;
- R₅ bedeutet:
- ein Wasserstoffatom;
- eine geradkettige oder verzweigte C₁₋₄-Alkylgruppe;
- eine geradkettige oder verzweigte C₁₋₆-Alkenylgruppe, die mit der Gruppe (R₆R₇N-)ₙ substituiert ist, wobei n 0 oder 1 bedeutet und wobei, wenn n = 1, R₆ und R₇ unabhängig voneinander ein Wasserstoffatom, eine geradkettige oder verzweigte C₁₋₆-Alkylgruppe bedeuten, die gegebenenfalls mit einer oder mehreren Gruppen substituiert ist, die unter den Gruppen Hydroxy, Amino, Halogen, vorzugsweise Chlor, Brom oder Fluor, oder C₁₋₃-Alkyl ausgewählt sind;
- R₈ bedeutet:
- ein Wasserstoffatom;
- eine geradkettige oder verzweigte C₁₋₆-Alkylgruppe und vorzugsweise Methyl oder Ethyl;
- L bedeutet eine zweiwertige Gruppe der folgenden Formeln L1 oder L2: 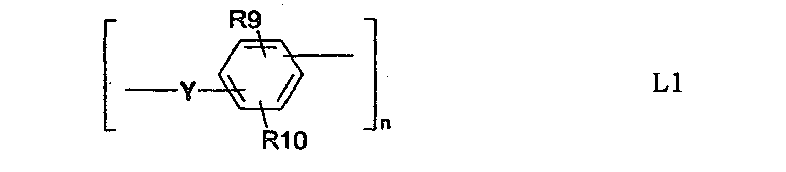 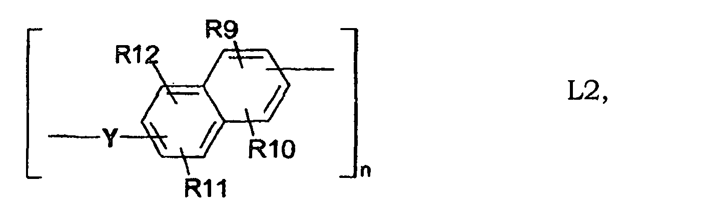
worin bedeuten:
- n 0 oder 1;
- R₉, R₁₀, R₁₁ oder R₁₂ unabhängig voneinander:
- ein Wasserstoffatom;
- eine geradkettige oder verzweigte C₁₋₆-Alkylgruppe, wobei diese Gruppe mit einer oder mehreren Gruppen substituiert sein kann, die unter den Gruppen Hydroxy, Amino, Halogen, vorzugsweise Chlor, Brom und Fluor, oder einer geradkettigen oder verzweigten C₁₋₃-Alkoxygruppe ausgewählt sein kann;
- eine geradkettige oder verzweigte C₁₋₄-Alkoxygruppe;
- eine Hydroxygruppe, Aminogruppe, ein Halogen oder eine geradkettige oder verzweigte C₁₋₃-Alkoxygruppe;
- eine Sulfonsäuregruppe;
- Y eine zweiwertige Gruppe, und
b) mindestens einen synthetischen Farbstoff, der von der Verbindung der Formel (I) verschieden ist.

2. Farbmittelzusammensetzung nach dem vorhergehenden Anspruch, **dadurch gekennzeichnet, dass** in der Formel (I) die Gruppe Y eine Alkylengruppe mit 1 bis 4 Kohlenstoffatomen, die gegebenenfalls an jedem Ende mit einer Carbonylgruppe oder einer Gruppe -CO-NH- oder -NH-CO- substituiert sein kann; eine Gruppe -CO-NH- oder -NH-CO-; eine Carbonylgruppe oder eine Azogruppe bedeutet.

3. Farbmittelzusammensetzung nach Anspruch 2, **dadurch gekennzeichnet, dass** n 1 bedeuten und Y -(CH₂)ₚ- bedeutet, wobei p eine ganze Zahl von 1 bis 4 ist.

4. Farbmittelzusammensetzung nach einem der vorhergehenden Ansprüche, **dadurch gekennzeichnet, dass** die Verbindung der Formel (I) einer Verbindung der folgenden Formel entspricht:

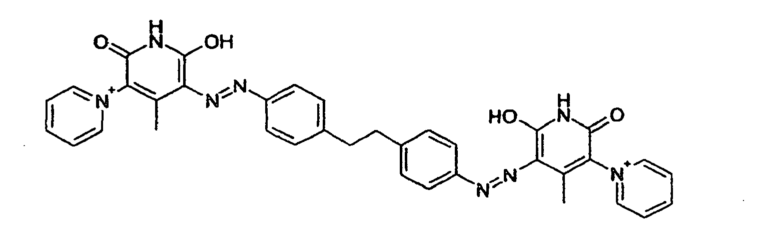

5. Farbmittelzusammensetzung nach einem der vorhergehenden Ansprüche, **dadurch gekennzeichnet, dass** der Mengenanteil der Verbindung der Formel (I) im Bereich von 0,01 bis 10 Gew.-%, bezogen auf das Gesamtgewicht der Farbmittelzusammensetzung, und vorzugsweise im Bereich von 0,005 bis 5 Gew.-%, bezogen auf das Gesamtgewicht der Farbmittelzusammensetzung, liegt.

6. Farbmittelzusammensetzung nach einem der vorhergehenden Ansprüche, **dadurch gekennzeichnet, dass** der synthetische Farbstoff, der von der Verbindung der Formel (I) verschieden ist, unter den nichtionischen, kationischen, anionischen, amphoteren oder zwitterionischen direktziehenden Farbstoffen oder deren Gemischen ausgewählt ist.

7. Farbmittelzusammensetzung nach dem vorhergehenden Anspruch, **dadurch gekennzeichnet, dass** der synthetische Direktfarbstoff unter den folgenden Farbstoffen ausgewählt ist: Acridin-Farbstoffen, Acridon-Farbstoffen, Anthranthron-Farbstoffen, Anthrapyrimidin-Farbstoffen, Anthrachinon-Farbstoffen, Azin-Farbstoffen, Azofarbstoffen, Azomethin-Farbstoffen, Benzanthron-Farbstoffen, Benzimidazol-Farbstoffen, Benzimidazolon-Farbstoffen, Benzindol-Farbstoffen, Benzoxazol-Farbstoffen, Benzopyran-Farbstoffen, Benzothiazol-Farbstoffen, Benzochinon-Farbstoffen, Bisazin-Farbstoffen, Bisisoindolin-Farbstoffen, Carboxanilid-Farbstoffen, Coumarinen, Cyaninen (Azacarbocyanin, Diazacarbocyanin, Diazahemicyanin, Hemicyanin, Tetraazacorbocyanin), Diazin-Farbstoffen, Diketopyrrolopyrrol-Farbstoffen, Dioxazin-Farbstoffen, Diphenylamin-Farbstoffen, Dithiazin-Farbstoffen, Flavanthron-Farbstoffen, Flavon-Farbstoffen, Fluorindin-Farbstoffen, Formazan-Farbstoffen, Hydrazon-Farbstoffen, Hydroxyketon-Farbstoffen, Indamin-Farbstoffen, Indanthron-Farbstoffen, Indigoiden, Indophenol-Farbstoffen, Indoanilin-Farbstoffen, Isoindolin-Farbstoffen, Isoindolinon-Farbstoffen, Isoviolanthron-Farbstoffen, Lacton-Farbstoffen, Methin-Farbstoffen, Naphthalimid-Farbstoffen, Naphthanilid-Farbstoffen, Naphtholactam-Farbstoffen, Naphthochinon-Farbstoffen, NitroFarbstoffen, Oxadiazol-Farbstoffen, Oxazin-Farbstoffen, Perilon-Farbstoffen, Perinon-Farbstoffen, Perylen-Farbstoffen, Phenazin-Farbstoffen, Phenothiazin-Farbstoffen, Phthalocyanin-Farbstoffen, Polyen/Carotinoid-Farbstoffen, Porphyrin-Farbstoffen, Pyranthron-Farbstoffen, Pyrazolanthron-Farbstoffen, Pyrazolon-Farbstoffen, Pyrimidinoanthron-Farbstoffen, Pyronin-Farbstoffen, Chinacridon-Farbstoffen, Chinolin-Farbstoffen, Chinonaphtholon-Farbstoffen, Squaran-Farbstoffen, Stilbenen, Styril-Farbstoffen, Tetrazolium-Farbstoffen, Thiazin-Farbstoffen, Thioindigo, Thiopyronin-Farbstoffen und Xanthen-Farbstoffen.

8. Farbmittelzusammensetzung nach einem der Ansprüche 6 oder 7, **dadurch gekennzeichnet, dass** der synthetische Direktfarbstoff unter den neutralen, sauren oder kationischen nitrierten direktziehenden Benzol-Farbstoffen, neutralen, sauren oder kationischen direktziehenden Azofarbstoffen, direktziehenden Chinon-Farbstoffen und insbesondere neutralen, sauren oder kationischen Anthrachinon-Farbstoffen, direktziehenden Azin-Farbstoffen, Triarylmethan-Farbstoffen, Indoamin-Farbstoffen, Methin-Farbstoffen, Styril-Farbstoffen, Porphyrin-Farbstoffen, Metalloporphyrin-Farbstoffen, Phthalocyanin-Farbstoffen, Methincyanin-Farbstoffen und fluoreszierenden Molekülen ausgewählt ist.

9. Farbmittelzusammensetzung nach einem der vorhergehenden Ansprüche, **dadurch gekennzeichnet, dass** der Mengenanteil des synthetischen direktziehenden Farbstoffes, der von der Verbindung der Formel (I) verschieden ist, im Bereich von 0,001 bis 10 Gew.-%, bezogen auf das Gesamtgewicht der Farbmittelzusammensetzung, und vorzugsweise im Bereich von 0,005 bis 5 Gew.-%, bezogen auf das Gesamtgewicht der Farbmittelzusammensetzung, liegt.

10. Farbmittelzusammensetzung nach einem der vorhergehenden Ansprüche, **dadurch gekennzeichnet, dass** der synthetische Farbstoff unter den Oxidationsbasen gegebenenfalls in Kombination mit mindestens einem Kuppler ausgewählt ist.

11. Farbmittelzusammensetzung nach dem vorhergehenden Anspruch, **dadurch gekennzeichnet, dass** die Oxidationsbase unter den p-Phenylendiaminen, Doppelbasen, p-Aminophenonlen, o-Aminophenolen und heterocyclischen Oxidationsbasen, einzeln oder in Form von Gemischen, sowie deren Additionssalzen mit einer Säure ausgewählt ist.

12. Farbmittelzusammensetzung nach einem der Ansprüche 10 oder 11, **dadurch gekennzeichnet, dass** der Mengenanteil der Oxidationsbase im Bereich von 0,0005 bis 12 Gew.-%, bezogen auf das Gesamtgewicht der Farbmittelzusammensetzung, und vorzugsweise 0,005 bis 8 Gew.-%, bezogen auf das Gesamtgewicht der Farbmittelzusammensetzung, liegt.

13. Farbmittelzusammensetzung nach einem der Ansprüche 10 bis 12, **dadurch gekennzeichnet, dass** der Kuppler unter den m-Phenylendiaminen, m-Aminophenolen, m-Dihydroxybenzolen, heterocyclischen Kupplern, einzeln oder in Form von Gemischen, sowie deren Additionssalzen mit einer Säure ausgewählt ist.

14. Farbmittelzusammensetzung nach einem der vorhergehenden Ansprüche, **dadurch gekennzeichnet, dass** sie ferner mindestens ein Oxidationsmittel enthält.

15. Farbmittelzusammensetzung nach dem vorhergehenden Anspruch, **dadurch gekennzeichnet, dass** das Oxidationsmittel unter Wasserstoffperoxid, Harnstoffperoxid, Alkalimetallbromaten oder Alkalimetallferricyaniden, Salzen von Persäuren, Redoxenzymen, wie Laccasen, Peroxidasen und Oxidoreduktasen (2 Elektronen) gegebenenfalls in Gegenwart ihres jeweiligen Donors ausgewählt ist.

16. Verfahren zum Färben von Keratinfasern und insbesondere menschlichen Keratinfasern, **dadurch gekennzeichnet, dass** die trockenen oder feuchten Keratinfasern mit einer Farbmittelzusammensetzung nach einem der Ansprüche 1 bis 15 während einer Zeitdauer in Kontakt gebracht werden, die ausreichend ist, um die gewünschte Färbung zu bilden.

17. Verfahren zum Färben nach dem vorhergehenden Anspruch, **dadurch gekennzeichnet, dass** die Zusammensetzung bedarfsgemäß vor der Anwendung durch Mischen einer Zusammensetzung, die mindestens eine Verbindung der Formel (I) und mindestens einen synthetischen Farbstoff, der von der Verbindung der Formel (I) verschieden ist, enthält, mit einer Zusammensetzung, die mindestens ein Oxidationsmittel enthält, hergestellt wird.

18. Verfahren zum Färben nach Anspruch 17, **dadurch gekennzeichnet, dass** die Zusammensetzung vor der Anwendung bedarfsgemäß durch Mischen einer Zusammensetzung, die mindestens eine Verbindung der Formel (I) enthält, einer Zusammensetzung, die mindestens einen synthetischen Farbstoff, der von der Verbindung der Formel (I) verschieden ist, enthält und einer Zusammensetzung, die mindestens ein Oxidationsmittel enthält, hergestellt wird.

19. Verfahren nach einem der Ansprüche 16 bis 18, **dadurch gekennzeichnet, dass** in beliebiger Reihenfolge die Farbmittelzusammensetzung(en) und die Zusammensetzung, die mindestens ein Oxidationsmittel enthält, aufgetragen werden.

20. Vorrichtungen mit mehreren Abteilungen zur Durchführung des Verfahrens nach einem der Ansprüche 16 bis 19, **dadurch gekennzeichnet, dass** sie eine Abteilung mit einer Farbmittelzusammensetzung, die mindestens eine Verbindung der Formel (I) und gegebenenfalls mindestens einen synthetischen Farbstoff, der von der Verbindung der Formel (I) verschieden ist, enthält; gegebenenfalls mindestens eine zweite Abteilung mit einer Farbmittelzusammensetzung, die mindestens einen synthetischen Farbstoff, der von der Verbindung der Formel (I) verschieden ist, enthält, und mindestens eine dritte Abteilung mit einer Zusammensetzung aufweist, die mindestens ein Oxidationsmittel enthält.
